Europäisches Patentamt

European Patent Office

Office européen des brevets

(11) Publication number: **0 149 751**
A2

(12) # EUROPEAN PATENT APPLICATION

(21) Application number: **84114035.3**

(22) Date of filing: **20.11.84**

(51) Int. Cl.⁴: **C 12 P 21/00**
C 07 K 15/04, C 12 N 5/00
//A61K37/02, (C12P21/00,
C12R1:91)

(30) Priority: **21.11.83 JP 218985/83**

(43) Date of publication of application:
**31.07.85 Bulletin 85/31**

(84) Designated Contracting States:
**AT BE CH DE FR GB IT LI NL SE**

(71) Applicant: **KYORIN PHARMACEUTICAL CO., LTD.**
**No. 5, Kanda Surugadai 2-chome**
**Chiyoda-ku Tokyo(JP)**

(72) Inventor: **Irikura, Tsutomu**
**No. 10-28, Ooizumigakuen-cho 7-chome**
**Nerima-ku Tokyo(JP)**

(72) Inventor: **Takagi, Koichi**
**No. 1691-6, Tsuchiya**
**Oomiya-shi Saitama-ken(JP)**

(72) Inventor: **Hosomi, Jiro**
**No. 186-20, Kitashimoarai Otone-machi**
**Kitasaitama-gun Saitama-ken(JP)**

(72) Inventor: **Murayama, Satoshi**
**No. 6095, Tomonuma Nogi-cho**
**Shimotsuga-gun Tochigi-ken(JP)**

(72) Inventor: **Saito, Koji**
**No. 6-8, Akatsuki 1-chome Oyama-shi**
**Tochigi-ken(JP)**

(72) Inventor: **Okazaki, Takashi**
**No. 5932, Aza Unoki Tomonuma Nogi-cho**
**Shimotsuga-gun Tochigi-ken(JP)**

(74) Representative: **Patentanwälte TER MEER - MÜLLER -**
**STEINMEISTER**
**Mauerkircherstrasse 45**
**D-8000 München 80(DE)**

(54) **Proteinaceous substance showing antitumorous action and method for its manufacture.**

(57) A proteinaceous substance having the following biochemical characteristics:

A) a strong cytolytic activity against mouse tumor cells L-929, but no cytotoxic activity for normal cells such as human fetus fibroblasts, human adult skin fibroblasts and chinese hamster fibroblasts (Don, V-79) and therefore a high tumor specificity; and

B) a strong anti-tumor and immunogenic activity against Meth-A sarcoma. When said substance is injected intravenously to the syngenic BALB/C mice inoculated with Meth-A sarcoma at a quantity of more than 72 units/head, not only hemorrhagic necrosis, but also complete degradation is observed. Thereafter, even if it is attempted again to transplant the Meth-A sarcoma cells under the skin of Mice cured completely, successfull plantation is not observed because of an acquired immunity;

C) the substance has no pyrogenetic action after intravenous injection to rabbits;

D) a molecular weight of 45,000 ± 5,000 (determined by the gel filtration method with Sephacryl S-200); and

E) an isoelectric point pl of 4.8 ± 0.3; and a method for its manufacture.

SPECIFICATION

**0149751**

1. Title of the Invention:
PROTEINACEOUS SUBSTANCE SHOWING ANTITUMOROUS ACTION AND
METHOD FOR ITS MANUFACTURE

2. Detailed Description of the Invention:
The present invention relates to a proteinaceous substance
having valuable biochemical characteristics and a method
for its manufacture. The substance of the present
invention is an antitumor-proteinaceous substance that
is released into the culture supernatant by the cultured
cells as shall follow; mammalian cultured cells that are
able to proliferate without any restriction and to
differentiate to macrophage-type by a lipopolysaccharide
of E. coli.  The cells take the form of round-type. The
cells have a phagocytic activity.

One of the matters having the closest relation to the
invention among the facts that were known before the
invention is a report by Pincus W.B. in 1967 (Pincus, W.B.,
J. Reticuloendothel. Soc. Vol. 4, pages 122-139 (1967)),
who described that when purified tuberculin protein was
added to the culture liquid of peritoneal cavity macrophage of normal guinea pigs, the macrophages released
into the medium a substance having a killing activity
against the tumor cells of mice. Until now, the tumor-
killing action of the macrophage has been considered
to be the cause of a direct contact with the target
cells. Therefore, this report documents an important
knowledge which shows that there lies a liquid intermediate substance.

Secondly, Meltzer M.S. et al made it clear in 1972
(Meltzer M.S., G.L. Bartlett, J. Natl. Cancer Inst.
Vol. 49, pages 1439-1443 (1975) that when purified
tuberculin protein was added to the mouse spleen cells

- 1 -

activated through the BCG sensitization and the cells were cultured, a substance was present in the supernatant liquid. The substance did not act against the normal fetal cells and the normal fibroblast cells, but did kill the L cells specifically. This report is also important to the point that the substance does differ from a indiscriminate cytotoxin (Lymphotoxin) obtained from lymphocyte and that it acts specifically to only the tumor cells.

Thirdly, Carswell et al made it clear in 1975 (Carswell E.A., L.J. Old et al, Proc. Natl. Acad. Sci., USA Vol. 72, pages 3666-3670 (1975) that when the colibacillus toxin LPS was injected intravenously to the mice infected by BCG, a substance having an activity of killing L cells was released in the serum 2 hr after the injection of LPS. Moreover, this serum did not show toxicity against the normal animals and the normal cells, but did induce the hemorrhagic necrosis or the complete cure after 0.5 ml of this serum was injected to mice beared Meth-A tumor. Carswell et al named the substance tumor necrosis factor (hereinafter abbreviated to as TNF). It was made clear that the molecular weight of TNF is about 150,000.

Afterwards, although many supplementary examinations were performed by investigators as Mathews N., Ruff M.R., Männel D.N., Kull F. C. Jr. et al, it has been obscure whether TNF is a pure substance or a mixture of contents of same activity.

Since Carswell et al studied TNF, the studies relating to active substance not active against the normal cells but active against tumor cells have been enhanced. The studies have been divided into four directions as follows:
1) The first direction of the investigation gave attention to find a lower toxic substance instead of LPS, such as Lipid A of low toxicity, Pseudomonas aeruginosa dead fungus, and endotoxid obtained by deacetylating LPS in the methanol were examined.

2) The second direction gave attention to the therapy of the human cancer by using TNF of mice and rabbits by the method of Carswell et al. The idea is based on that interleukin-1, interleukin-2 which are a kind of lymphokines as TNF have also a property to act beyond the difference of species, and that there is a possibility to be able to apply TNF obtained from other animals to human beings. However, this method has a difficulty in that the extraction should be done from the whole serum. Therefore, quite different results are shown according to the investigators even if only taking the molecular weight into consideration.

3) The third way tried to obtain the antitumorous active substance from the supernatant liquid of the culture cells in vitro. It is similar to 2) in that this substance may be able to be applied to human beings even if obtained from the cells of animals so that an active substance originating from human beings is obtainable.

4) The fourth direction relates to a method to obtain by a technique of so-called biotechnology, the antitumorous active substance from the culture cells, wherein the messenger RNA of the cells is isolated and transformed into the colibacillus or the yeast. In the case of this method, a peptide chain can be obtained, but there is the possibility not to reach to the immuno-tolerant state because of the lack of sugar chain. Therefore, this method remains doubtful.

Now, in the case of the method for obtaining the antitumorous active substance from the supernatant liquid of the culture as described in 3), the growth of the cells obtained from the normal animals is slow, and it is difficult to culture in succession over perdurable generation  so this is unsuitable for an industrial method. It is conceivable naturally to use the cells as the tumorous cells which are fast in the proliferation. The investigators who used these for the first time are Männel D.N. et al (Männel D.N., R.N. Moore and S.E. Marzenhagen, Infect. and Immum., Vol. 30, pages 523-530 (1980)). They reported that, when LPS was added to macrophage-like cell  PU5-1.8 and the cells were cultured for 2 hours, the cells released into the medium a substance having a heat stability for 30 minutes at 56°C and molecular weight of 50,000 to 60,000.

Moreover, coming to 1983, Williamson B.D. et al (Williamson B.D., E.A. Carswell, B.Y. Rubin, J. S. Prendergastand, L.J. Old, Proc. Natl. Acad. Soc. USA, Vol. 80, pages 5397-5401 (1983) found that human TNF was produced from the human B cell tumor transformed by the Epstein bar virus and the molecular weight of it was about 70,000, Ohnishi et al (Vienna Intern. Chemotherapy Soc., Preprint PS 12.4 : 7-26 (1983) also reported that human B tumor cells, such as BALL-1 cells released an antitumorous substance having a molecular weight of about 15,000 when the cells were treated with Sendai virus.

The object of the present invention therefore is to provide a proteinaceous substance having valuable biochemical characteristics, such as a strong anti-tumor and immunogenic activity, and a method for its manufacture.

According to a first embodiment the present invention relates to a proteinaceous substance characterized by the following biochemical characteristics:

A) a strong cytolytic activity against mouse tumor cells L-929, but no cytotoxic activity for normal cells such as human fetus fibroblasts, human adult skin fibroblasts and chinese hamster fibroblasts (Don, V-79), and therefore, a high tumor specificity;

B) a strong anti-tumor and immunogenic activity against Meth-A sarcoma;

C) no pyrogenetic action after intravenous injection to rabbits;

D) a molecular weight of 45,000 $\pm$ 5,000 (determined by the gel filtration method using Sephacryl S-200); and

E) an isoelectric point pI of 4.8 $\pm$ 0.3.

A second embodiment of this invention covers a method for the manufacture of the above proteinaceous substance which is characterized by allowing to proliferate in the complete growth medium containing calf fetal serum among mammalian tumor cultured cells, only the histocyte-type cells showing phagocytic action, then culturing said cells in the serum-free medium in order to obtain the supernatant liquid of the culture containing said proteinaceous substance and isolating said proteinaceous substance from said supernatant liquid.

The further subclaims 3 to 12 relate to preferred embodiments of this second aspect of this invention, by specifically using among the reticulosarcoma-type cultured cells, cloned J 774.1 cells or the cultured cloned round histocyte-type cells having a phagocytic ability, derived from human lymph nodes such as U-937, RS-2, RS-3, MC-8-3, MC-12, RPMI 8866, ARH-77, Wa and KCCL cells or the cultured cloned round cells having phagocytic ability derived from tumor cells of human myeloma such as K-562, D-98 and OST cells or the cloned histocyte cells having phagocytic ability derived from human Burkitt lymphoma such as EB-3, Raji, and P3HR-1 cells or the hybridoma cells between the U-937 cells and the human B cells such as BALL-1 cells or mouse lymphoma cells having a phagocytic action, such as RAW 264, IC-21, P 388D1 and PU5-1.8 cells and/or by promoting the release of said proteinaceous substance by adding a trace of 2-mercaptoethanol or a trace of phorbol myristate acetate (hereinafter abbreviated as PMA) to the culture cells used.

According to another preferred embodiment of this method the supernatant liquid of the culture in the serum-free medium is precipitated with ammonium sulfate and submitted to gel filtration with Sephacryl S-200, collecting the portion not absorbed by Blue Sepharose and passing it further through DEAE Sephadex A-50, and if desired subjecting it to HPLC with TSK G-3000 SWG and/or with TSK DEAE-3SW either.

Preferably monoclonal antibodies are obtained from the hybridoma between spleen cells and mouse myeloma cells such as P3-X63-Ag. 8.653 by injecting the proteinaceous substance defined above into the skin of the mice together with an adjuvant and further performing the intensive immunity.

Finally it is possible to allow the monoclonal antibodies to bond with a suitable carrier for further purification or quantitative determination such as an ordinary radioactive substance, peroxidase, or the like.

The differences between the substance of the invention and the substance in the reports indicated above is as follows:

A)     Only the histocytes having a phagocytic among the reticulosarcoma were cloned and used. Among J774.1 cells cloned by Ralph P. et al (Ralph P. and L. Nakoinz, Nature Vol. 257, page 393-394 (1975)) only the circular cells were further cloned and used. Moreover, the human cells of a similar series, U-937 etc., are usable after having been submitted to the cloning for obtaining cells which are circular and have a strong phagocytic ability.

Besides, Burkitt lymphoma cells transformed with Epstein bar virus such as Raji, DAUD 1, RAMOS can be used after submitted to the cloning. Furtheron, it is possible to make use of the hybridoma with the monocytes such as U-937 or J-111 and the B cell tumor, for exmaple, BALL-1, U-704 and the like.

It is a special feature to use respective cells without allowing to differentiate to the macrophage with the stimulant.

B)    Although the cells assumed the form of monocytes and described in A) are able to differentiate to the macrophage-like cells by adding LPS, vegetable lectin and the like, the special feature of the present invention lies in that it is released from the cells having a shape before differentiation.    Therefore, the extraction is easy since absence of substance such as LPS which is difficult to separate.

C)    The culture cells shown in A) grow vigorously in a medium containing calf fetus serum (doubling times, 24 hours).    After allowed to proliferate sufficiently with this calf fetal serum, the medium is exchanged to non -serum medium and the cells are cultured for several days to collect the supernatant liquid.

The invention has a special feature in that since the cells remained behind are not received any damage, they are

utilized for again producing the supernatant liquid of the non-serum medium after they were transferred to the serum medium.    This is extremely advantageous industrially.

D)    On the extraction of the proteinaceous substance of the invention from the serum-free medium, bovine albumin and transferrin of the main proteins in the medium can be briefly removed with Blue Sepharose and DEAE-Sephadex.

Moreover, ammonium sulfate does not affect on the
purification of the substances of the invention because it
causes neither the polymerization nor the association.
Therefore, pure substance can be obtained easily.

Even if lysozyme or interleukin-1 are present in the
supernatant liquid obtained by the method of the invention,
they can be removed briefly by the use of gel filtration or
ultrafiltration, since they are molecular weight of 14,400
and 15,000 in either case.

E)    The substance of the invention is injected at
random into the skin of mouse together with adjuvant and
further allowed to sensitize once more, and collect the
spleen cells.

Then, the hybridoma with the B tumor cell of syngenic
mice is prepared to obtain a monoclonal antibody.

If this method utlizes to the purification and the
determination, the process control can be done with great
ease.

F)    The proteinaceous substance obtainable by the method
of the invention has a molecular weight of 45,000 $\pm$ 5,000
and an isoelectric point of pI 4.8 $\pm$ 0.3.

It is stable for heating at 56°C for 30 minutes.  There are
different property from human TNF obtained from the B
cells of human by Williamson B. D. et al since their TNF is
a molecular weight of 70,000.   Further, this is different
from the active substance obtained from BALL-1 by Ohnishi
H. et al which has a molecular weight of 15,000.

Therefore, the physiochemical value of this substance are different in both case.

G)    The substance of the invention has not a pyrogenetic activity, and it shows neither the abnormal behavior nor the death even if the intravenous injection is made to rabbits large quantity.

H)    The substance is different in the physiological action from Colony stimulating factor (CSF) which shows the action to increase the granulocytes.    CSF is made by adding a stimulator such as LPS, dextran, zymosan, tuberculin, PMA and the like in the macrophage culture.

I)    When allowed to react PMA or LPS with P 388D1 cells (cultured cells of macorophage-type), interleukin-1 is formed in the supernatant liquid of the culture.    Since this has a molecular weight of 14,000 and has pyrogenetic activity or it put out an amyloid-like substance in the serum, these properties are different evidently from the proteinaceous substance according to the invention.

Besides, properties approximate to the antitumorous active substances originating from the cells known hitherto are as follows.

J)    The substance of the invention shows a cell-killing effect on L-929, Meth-A, S-180 and L 1210 of the tumor cells of mouse in vitro.    At the same time, this shows a similar killing effect on the carcinoma of human (HSC-1, EAC-1 and HeLa).

But, this shows no killing effect on the normal cells such as the fibroblast of skin of adult or fetus of human, and V-79 and Don cells of animals.

That is, its tumor specificity is high.

K)     When the diameter of the congeneitic Meth-A tumor reached to 5 to 6 mm in the BALB/c mice, the mice subjected to the intravenous injection with the compound of the invention once at 72 units.    As a result, it was observed not only the tumor falls into a hemorrhagic necrosis, but also the infiltration of the lymphocites.

Afterward, the tumor drops out after about 7 days and 84% of animals are·cured completely.     The rate of the complete cure is high compared with 25% in the cases of carswell et al.

When 10  cells of the same tumor are planted again under the skin of completely cured mice, the tumor is not planted alive.   This result shows the formation of the immunity to the tumor.   Hereinafter, the proteinaceous substabce in accordance with the invention is called antitumorous factor (hereinafter abbreviated to as ATF).

L)     ATF is an acidic protein having a molecular weight of 45,000 ± 5,000 and it may be involve some sugar.

It is sensitive with trypsin, but stable in the heating at 56°C for 30 minutes.    It is not inactived with RNAase and also with galactosidase.

     In following, the invention will be explained in more detail using concrete examples.

Experiment 1

Measuring method of the cytotoxic activity

The activity unit of ATF was measured using mouse tumor
L cells as a target cells.  Namely, L cells were suspended
at the concentration of $2 \times 10^5$ cells/ml of the Eagle's
minimum  essential medium (MEM) supplemented with 10% Fetal
calf serum (FCS, heat-inactivated) and 0.1 ml of this
suspension was added to each well of the 96-well microplate
and the cells were cultured at 37°C in the 5% $CO_2$ incubator.
After the incubation of 24 hours, the medium was removed
and exchanged for the medium containing serial four
-dilution of ATF, and the cells were further cultured
for 48 hours.  The number of aliving cells was measured by
a hemocytometer after staining with erythrosin.
The titer was calculated by the reciprocal of the dilutions
of ATF having 50% killing of control.

Experiment 2

In vitro cytotoxic activity of ATF on the tumor cells
and normal cells was measured using the medium obtained
from Example 1.   The magnification of dilution corres-
ponding to 50% killing on the human lung squamous cell
carcinoma  (EAC-1) and human skin squamous cells carcinoma
(HSC-1) was 100 and 32 times, respectively.

Otherhand, the cell killing on the normal cells of
human diploid fibroblast (IMR-90) and the chinese hamster
Don cells, was not observed, even if treated with the
medium just as it was.

Experiment 3

The pyrogenetic action of ATF

For the purpose of comparing with interleukin-1, the medium (10 ml) in Example 1 A was injected into the vein of New Zealand White rabbit and the body temperature of the rectum was measured.  As the result, the rise of temperature was recognized only 0.3°C at 1 hour after injection and 0.1°C at 2 hours.  Therefore, the substance of the invention (ATF) has no pyrogenetic action.

Experiment 4.

In vivo anti-tumor activity of ATF

$10^6$ cells of Meth-A fibrosarcoma were transplanted under the skin at the inguinal position of BALB/c inbred mice. After 7 days, the mice were injected intraveneously with 0.2 ml (specific activity, 90 units/mg protein) of the sample which had been prepared by concentrating the medium obtained from Example 1 A).

On next day of the injection, the hemorrhagic necrosis having diameter of 2 to 3 mm was observed for the individual of 17 mice in 19 mice (89%) as shown in Photo. 1.  The tumor portion of one of them was excised in order to investigate the histopathological change of the periphery of tumor tissue or the tumor.

Many lymphocyte-like cells which is suspected to originate from the host were recognized in the periphery of the tumor tissue, as shown in Photo. 2.

Thereafter, the size of Meth-A sarcoma decreased with increasing the time of breeding, especially the individual caused the tumor necrosis was remarkable.

On the 7 days after injection, the formation of crust behind the degradation of tumor was observed.    On the 10 days, the crust peeled off, as shown in Photo. 3.

Such complete disappearance of the tumor was recognized for 16 mice in 19 mice (84%).    Although Meth-A cells were transplanted again under the skin of the individuals having been healed completely, they were not grown alive.    This result indicates the existence of an acquired immunity.

Experiment 5.

Effect of ammonium sulfate on the purificatin of ATF Both of the supernatant liquid of Exmaple 2 and the 0-65% saturated ammonium sulfate fraction described in Example 5 were applied to Sephacryl S-200 (Pharmacia fine chem.) column (2.5 x 85 cm) and the difference of the molecular weight was investigated for both samples. 0.02 M phosphate buffer containing 0.03 M sodium chloride (pH 7.5) was used to equilibrate the column and to elute ATF.    The difference in elution volume was hardly seen for both sample.

From this result, it is not considered that the fractionation with ammonium sulfate induce the association or the binding with other protein.

Experiment 6.

The determination of molecular weight of ATF

Partially purified ATF was applied to Sephacryl S-200 (Parmacia fine chem.) column (2.5 x 85 cm) equilibrated with 0.02 M phosphate buffer containing 0.3 M sodium chloride (pH 7.5).     The molecular weight of ATF was determined from the elution volume of ATF fractions having the activity of L cell killing.     The column was calibrated with ribonuclease A, chymotrypsinogen A, ovalubumin, borine serum albumin, blue dextran as m.w. markers.     As a result, the molelular weight was found to be 45,000 $\pm$ 5,000.

Experiment 7.

The determination of isoelectric point of ATF

The isoelectric point was measured by polyacrylamide disc gel electrophoresis used the reagent Ampholine (pH range 3.5-7.0) (made by LKB Co.). Gel was cut at intervals of 5 mm, extracted with distilled water, and the L cell killing activity and pH of each extracted fractions was measured.

As a result, the isoelectric point of ATF was found to be pI 4.8 $\pm$ 0.3.

Example 1.

A)    ATF production by complete growth medium

$3 \times 10^{6}$ cells of J774.1 were inoculated in the RPMI -1640 medium supplemented with 20% fetal calf serum (FCS), and cultured at 37°C in the 5% $CO_2$ incubator.    Many cells taked the form of sphere or monolayer and sticked firmly to the bottom of dish and prolifered vigorously.

When the floating cells began to appear, J774.1 cells were plated to new dishes at dilution of 10:1 after harvesting with a rubber-pliceman from confluent cultures.

During this time, cultured supernatant was collected every 3 to 4 days, the supernatant liquid containing the substance of the invention was obtained. By this method, the supernatant liquid having the titer of 500 units/ml was obtained.

B) The L cell-killing ability (titer) was increased with inceasing plate number of cells or cultured day.

When J774.1 cells were cultured at a density of $1.4 \times 10^5$ cells/cm$^2$ in the same medium as in Example 1, the titer increased to 140 units/ml on 3 days and to 630 units/ml on 6 days. Also, at a density of $10.7 \times 10^4$ cells/cm$^2$, the titer increased to 630 units/ml on next day and to 1,580 units/ml on 4 days.

Example 2.

ATF production by serum-free medium

After J774.1 cells were grown confluently in RPMI-1640 medium containing fetal calf serum as in Example 1, the culture medium was exchanged for serum-free medium and then J774.1 cells were further cultured for several days. As the result, the supernatant liquid having the titer of 2,000 units/ml was obtained.

Example 3.

ATF production by roller culture method

J774.1 cells were cultured at 37°C in the serum-free medium for 3 to 7 days by using a roller culture bottle, and the supernatant liquid containing the titer of 1,412 units/ml was obtained.

Example 4.

Mass culture by microcarrier

The J774.1 cells and the microcarrier such as DEAE
-dextran beads (Dainihon Seiyaku) or Cytodex (made by
Pharmacia Co.) were added in a spinner flask containing the
RPMI-1640 medium supplement with 20% FCS.
The culture was carried out for 3 days at 37°C to allow
the J774.1 cells to attach to the microcarrier.
After the culture medium was exchanged the medium
containing serum for the serum-free medium and the
culture was further continued at 37°C.        By this method,
the supernatant liquid containing 700 units/ml was
obtained.

Example 5.

Purification from the serum-free medium

70 ml of 0.2 M phosphate buffer containing 1 M sodium
chloride (pH 7.5) was added to 3 liter of supernatant
obtained in Example 2, the mixture were atirred in the
ice, and the pulverized ammonium sulfate was added to
the mixture in order to saturate 65%.
After 4 hours, the mixture was centrifuged (12,000 r.p.m.,
30 minutes), and the precipitate was dissolved into
1.5 ml of 0.2 M phosphate buffer containing 1 M sodium
chloride (pH 7.5).
At this step, the recovery of the activity was 95% and
the degree of the purification was 2.2 times of the
supernatant of the culture.

Then, the ammonium sulfate fraction was applied to Sephacryl S-200 column (2.5 x 85 cm) which had been equilibrated previously with 0.02 M phosphate buffer containing 0.1 M sodium chloride (pH 7.5).

Elution was performed with same buffer at a flow rate of 20 ml/hr.        9.7 ml fractions were collected continuously.  Fractions with ATF activity were pooled and concentrated with ultrafiltration.

At this step, the recovery of the activity was 99% and the degree of the purification was 3.2 times.

Through the whole steps, the recovery of the activity was 94% and the degfree of the purification was 7.2 times.

Next, the active fraction obtained by the gel filtration was passed through a Blue-Sepharose CL-6B (Pharmacia fine Chem.) column (2.0 x 11 cm) which had been equilibrated previously with 0.01 M Phosphate buffer containing 0.05 M sodium chloride (pH 7.5).

Flow rate 4 ml/hr and 6 ml fractions were collected.

After washed sufficiently with the same buffer, the absorved substance was eluted with 0.01 M phosphate buffer containing 1.0 M sodium chloride (pH 7.5).

The absorbed fraction had not the ATF activity, and all of the activity were recovered in the non-adsorbed fraction.

The active fraction was concentrated by ultrafiltration.

At this step, the recovery of the activity was 84% and the degree of the purification was 12 times.    Through the whole steps, the recovery of the activity was 78 % and the degree of the purification was 85 times.

Next, the active fraction was loaded onto DEAE-Sephadex A-50 (Pharmacia fine Chem.) column (2.0 x 8.0 cm) which had been equilibrated previously with 0.02 M phosphate buffer containing 0.1 M sodium chloride (pH 7.5). After washing with the same buffer, elution was conducted with 0.02 M phosphate buffer containing 0.3 M sodium chloride and that containing 0.5 M sodium chloride, respectively.

The active fraction was eluted with 0.02 M phosphate buffer containing 0.3 M sodium chloride.   The active fraction was concentrated by ultrafiltration.

At this steps, the recovery of the activity was 80 % and the degree of the purification was 5 times. Through the whole steps, the recovery of the activity was 63% and the degree of the purification was 427 times.

The active fraction was loaded onto HPLC with TSK-G3000 SWG (2.1 ID x 60 cm TOYOSODA CO., LTD.) which had been equilibrated with 0.01 M phosphate buffer containing 0.3 M sodium sulfate pH 6.8.   ATF activity was eluted with same buffer.   2 milliliter fractions were collected at a flow rate of 2.0 ml/min.

At this steps, the recovery of the activity was 37% and degree of the purification was 2.4 times.   Through the whole stage, the recovery of the activity was 23% and the degree of the purification was 1025 times.

The active fraction of TSK-G3000 SWG was loaded onto HPLC with TSK-DEAE-3SW (7.5 ID x 75 mm TOYOSODA CO., LTD) which had been equilibrated with 0.04 M phosphate buffer pH 6.8.     The column was washed with same buffer and that
containing 0.15 M sodium chloride respectively.     A linear
salt gradient from 0.15 M to 0.5 M sodium chloride was used
to elute ATF activity.    1.2 milliliter fractions were
collected at a flow rate of 0.6 ml/min.    This purified ATF
showed single band on polyacrylamide slab gel electrophoresis.

At this stage, the recovery of the activity was 30% and
the degree of the purification was 2.5 times.
Through the whole stage, the recovery of the activity was
7% and the degree of the purification was 2563 times.
The specific activity was 1.1 x 10$^6$ units/mg protein.

Example 6.

Purification from the serum-containing medium

250 ml of 0.2 M phosphate buffer containing 1 M sodium
chloride (pH 7.5) was added to 3 liter of supernatants
obtained in Example 1.    The pulverized ammonium sulfate
was gradually added to the mixture under stirring in the
ice in order to saturate 30%.    After 3 hours, the
mixture was centrifuged at 12,000 r.p.m. for 30 minutes and
the supernatant was collected.    Then, the pulverized
ammonium sulfate was gradually added to the supernatant
under stirring in ice in order to saturate 65%.
After 4 hours, the mixture was centrifuged at 12,000 r.p.m.
for 30 minutes, and the precipitate was dissolved into
small amounts of 0.02 M phosphate buffer containing 0.1 M
sodium chloride (pH 7.5).

At this step, the recovery of the activity was 98% and the degree of the purification was 1.6 times of the supernatant of the culture.

Then, this 30-65% fraction was dialyzed overnight with 0.02 M phosphate buffer containing 0.1 M sodium chloride (pH 7.5) and loaded onto DEAE-Sephadex A-50 column (9 x 15 cm) which had been equilibrated previously with the same buffer.  After washing with the same buffer, the elution was conducted with 0.02 M phosphate buffer containing respecive concentrations of 0.2 M, 0.3 M and 0.5 M sodium chloride (pH 7.5).   The fractionation was conducted each 15 ml at flow rate of 60 ml/hr and the active fractions was concentrated by ultrafiltration.

At this step, the recovery of the activity was 93% and the degree of the purification was 38 times.   Through the whole steps, the recovery of the activity was 72% and the degree of the purification was 60 times.

Next, the the active fraction obtained as above was applied to Sephacryl S-200 column (2.5 x 85 cm) which had been equilibrated previously with 0.02 M phosphate buffer containing 0.1 M sodium chloride (pH 7.5).
The fractionation was conducted each 6 ml at floew rate of 20 ml/hr.

At this step, the recovery of the activity was 95% and the degree of the purification was 12 times.   Through the whole steps, the recovery of the activity was 68% and the degree of the purificatin was 725 times.

Next, the active fraction was concentrated by ultrafiltration and passed through a Blue-Sepharose CL-6B column (5 x 10 cm) which had been equilibrated previously with 0.01 M phospahte buffer containing 0.05 M sodium chloride (pH 7.5).    The absorbed fraction was eluted with 0.01 M phosphate buffer solution containing 1.0 M sodium chloride (pH 7.5).    The activity was observed in the non-absorbed fraction.    The active fraction was concentrated.

At this step, the recovery of the activity was 82% and the degree of the purification was 6 times.    Through the whole step, the recovery of the activity was 56% and the degree of the purification was 4,320 times.

The active fraction was loaded onto HPLC with TSK-G3000SWG

(2.1ID x 60 cm TOYOSODA CO., LTD.) which had been equilibrated with 0.01 M phosphate buffer containing 0.3 M sodium sulfate pH 6.8.    ATF activity was eluted with same buffer.    2 milliliter fractions were collected at a flow rate of 2.0 ml/min.

At this step, the recovery of the activity was 35% and degree of the purification was 4 times.    Through the whole stage, the recovery of the activity was 20% and the degree of the purification was 17280 times.

The active fraction of TSK-G3000SWG was loaded onto HPLC with TSK-DEAE-3SW (7.5ID x 75 mm TOYOSODA CO., LTD.) which had been equilibrated with 0.04 M phosphate buffer ph 6.8.

The column was washed with same buffer and that containing 0.15 M sodium chloride respectively.    A linear salt gradient from 0.15 M to 0.5 M sodium chloride was used to elute ATF activity.     1.2 milliliter fractions were collected at a flow rate of 0.6 ml/min.    This purified ATF showed single band on polyacrylamide slab gel electrophoresis.

At this step, the recovery of the activity was 28% and the degree of the pulification was 3 times.
Through the whole stage, the recovery of the activity was 6% and the degree of the purification was 51840 times. The specific activity was $1.0 \times 10^6$ units/mg protein.

Example 7.

Purification of ATF with monoclonal antibody

1)   Production of monoclonal hybridoma

ATF purified partially is injected subcutaneously to the C3H/HeN mice (other species, if necessary) together with adjuvant.    After the immunity of once a week was carried out 3 to 4 time.    For the mice showing a high titer ATF, is injected intraperitoneally without adjuvant, and 3 days later, the mice are killed and the spleen was removed.    To prepare the spleen cells, the spleen is loosened to piesces by a pincettes and removed the erythrocytes with hemolysis buffer.    The spleen cellls are mixed with the myeloma cells of mouse (P3-x 63-Ag 8.653 or P3 NS-1) at a ratio of 10:1 to 1:1.    The cells of both kinds are fused with polyethylene glycol. The fused cells are cultured in the HAT selection medium during about 2 weeks, and they are further cultured in the HT medium removing the aminopterin from the HAT medium.

The supernatant of the fused cells culture and ATF solution diluted appropriately is added to the L cells culture at the same time. The fused cells having an ability to neutralize the activity of ATF are selected, it is regarded that the cells are hybridoma producing the antibody to ATF.    The cloning of hybridoma is carried out by the limiting dilution method.    The cloning cells are submitted to screening again at a time when the number of the cells is proliferated to an appropriate amount, and the positive ones are
submitted to cloning again.

2)    Mass production of the monoclonal antibody and purification

At first, we select the clone which has the
highest activity among the established hybridoma clones
producing anti-ATF antibody.    The clone is cultured on
a large scale to produce a large amount of the antibody.
There are two ways for the method of large scale culture.
One is a method in which 10  cells are intraperitoneally injected to mice, and allow to proliferate as the ascites cancer, and the other is a method in which the cells
are cultured in a large flask using the serum-free medium.
After concentrating by using ammonium sulfate or
ultrafiltration the ascites or the supernatant liquid of
the culture, the immunoglobulin is removed by passing
through the ion-exchange resin such as DEAE-Sephadex etc.
or proteinA-cellulose.

3)  Purification of ATF with the affinity column

Purified immuno-globulin is bonded to the carriers
such as Agarose and Sepharose activated with CNBr, and
this is filled in a column having appropriate volume.
After allowed to bind only ATF by passing the culture
supernatant liquid of the massive culture of J 774.1 cells
through this column, it is eluted by raising the ionic
strength to obtain pure ATF.

4)    Determination with the labelled antibody

**0149751**

By labelling I to the monoclonal antibody obtained with an ordinary radioactive substance, peroxidase or the like, the content of ATF can be quantitatively determined by a color development method faster than the measurement of activity by the biological method.

3. Brief Description of the Drawings

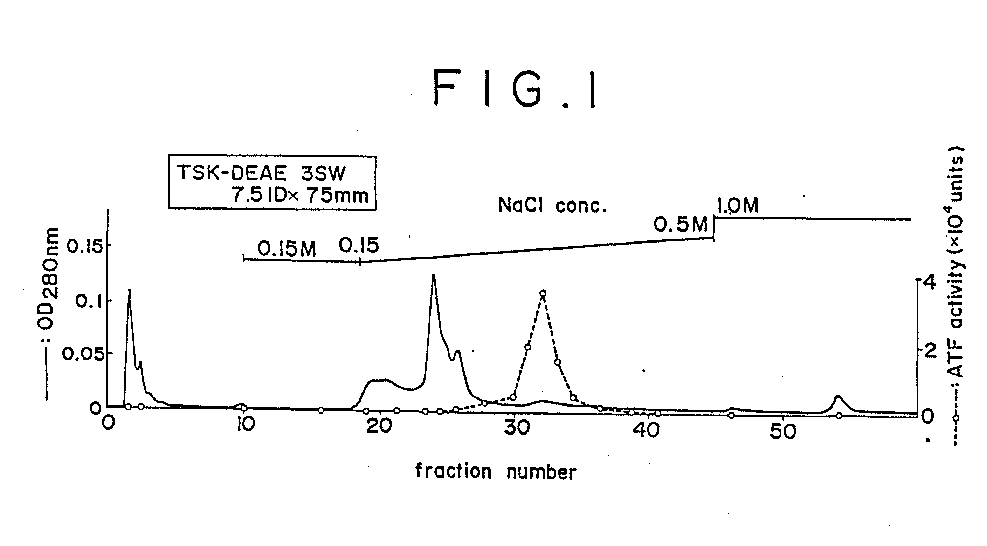

Fig. 1    shows the elution profile of ATF by HPLC with TSK-DEAE 3SW column. ATF fraction on a TSK-G3000SWG column in Experiment 5 was purified by TSK-DEAE 3SW column. 1.2 ml fractions were collected at a flow rate of 0.6 ml/min.

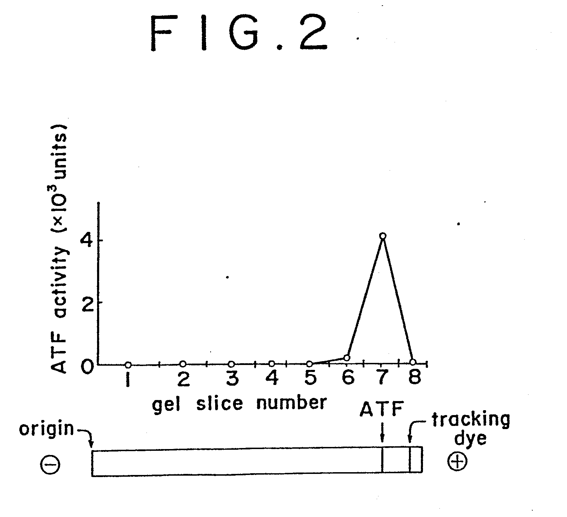

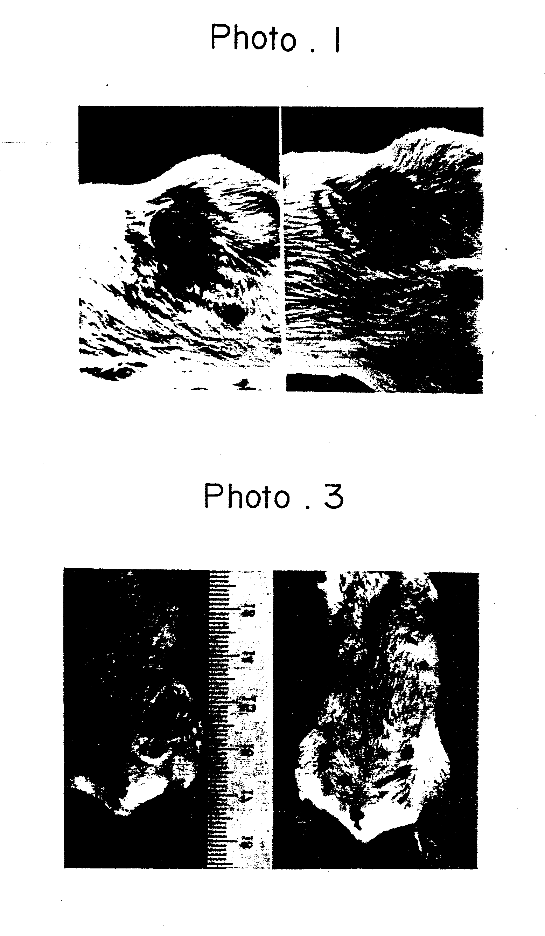

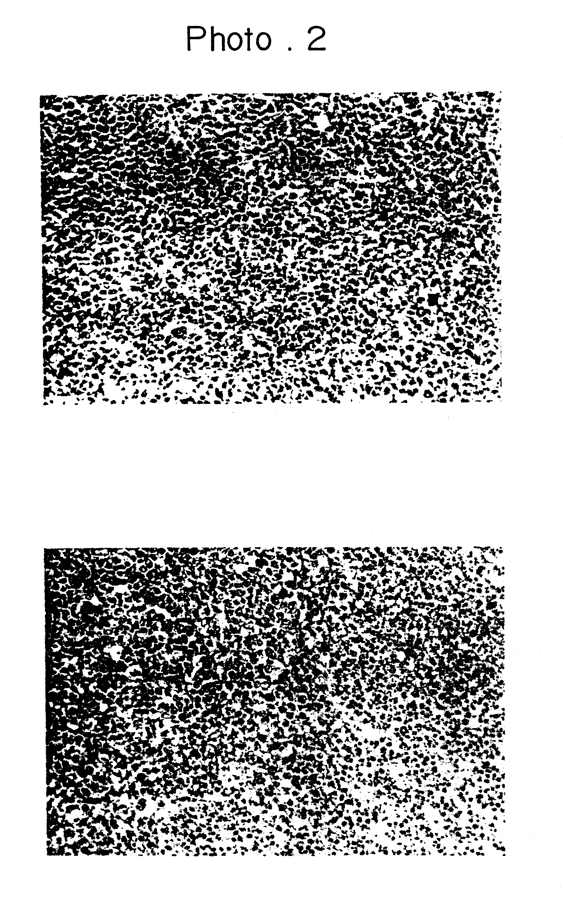

Fig. 2    shows the polyacrylamide gel electrophoretic profiles of purified ATF. Gel was stained by coomassie Brilliant Blue. Other gel was cut at intervals of 5 mm, extracted with RPMI-1640 medium and ATF activity was assayed.

Photo 1    shows the hemorrhagic necrosis of mouse shown in experiment 4. The left is a control animal and the right shows the hemorrhagic necrosis at 24 hours after injected with the supernatant in Example 1 A).

Photo 2    is the histological preparation of Meth-A tumor tissue in Experiment 4. The upper is that of Meth-A tumor of control mice. The lower is that of the animal in which the supernatant obtained from Example 1 A) was injected. In the lower, it is seen that the lymphocyte-like cells are infiltrating around the tumor cells showing the destruction of cells.

Photo 3    shows an example of the complete heal shown in Experiment 4. The left is control animal and the right is a mouse subjected to the injection with the supernatant liquid in Example 1 A).

## Claims

1. A proteinaceous substance characterized by the following biochemical characteristics:

   A) a strong cytolytic activity against mouse tumor cells L-929, but no cytotoxic activity for normal cells such as human fetus fibroblasts, human adult skin fibroblasts and chinese hamster fibroblasts (Don,V-79), and therefore, a high tumor specificity;

   B) a strong anti-tumor and immunogenic activity against Meth-A sarcoma;

   C) no pyrogenetic action after intravenous injection to rabbits;

   D) a molecular weight of 45,000 $\pm$ 5,000 (determined by the gel filtration method using Sephacryl S-200); and

   E) an isoelectric point pI of 4.8 $\pm$ 0.3.

2. A method for the manufacture of a proteinaceous substance having the following biochemical characteristics:

   A) a strong cytolytic activity against mouse tumor cells L-929, but no cytotoxic activity for normal cells such as human fetus fibroblasts, human adult skin fibroblasts and chinese hamster fibroblasts (Don,V-79), and therefore, a high tumor specificity;

   B) a strong anti-tumor and immunogenic activity against Meth-A sarcoma;

   C) no pyrogenetic action after intravenous injection to rabbits;

   D) a molecular weight of 45,000 $\pm$ 5,000 (determined by the gel filtration method using Sephacryl S-200); and

   E) an isoelectric point pI of 4.8 $\pm$ 0.3;

characterized by allowing to proliferate in the complete growth medium containing calf fetal serum among mammalian tumor cultured cells, only the histocyte-type cells showing phagocytic action, then culturing said cells in the serum-free medium in order to obtain the supernatant liquid of the culture containing said proteinaceous substance and isolating said proteinaceous substance from said supernatant liquid.

3. The method according to claim 2 characterized by using among the reticulosarcoma-type cultured cells, cloned J 774.1 cells.

4. The method according to claim 2, characterized by using the cultured cloned round histocyte-type cells having a phagocytic ability, derived from human lymph nodes such as U-937, RS-2, RS-3, MC-8-3, MC-12, RPMI 8866, ARH-77, Wa and KCCL cells.

5. The method according to claim 2, characterized by using the cultured cloned round cells having phagocytic ability derived from tumor cells of human myeloma such as K-562, D-98 and OST cells.

6. The method according to claim 2, characterized by using the cloned histocyte cells having phagocytic ability derived from human Burkitt lymphoma such as EB-3, Raji, and P3HR-1 cells.

7. The method according to claim 2, characterized by using the hybridoma cells between the U-937 cells and the human B cells such as BALL-1 cells.

8. The method according to claim 2, characterized by using mouse lymphoma cells having a phagocytic action, such as RAW 264, IC-21, P 388D1 and PU5-1.8 cells.

9. The method according to claims 2 to 8 characterized by promoting the release of said proteinaceous substance by adding a trace of 2-mercaptoethanol or a trace of phorbol myristate acetate (hereinafter abbreviated as PMA) to the culture cells used.

10. The method according to claims 2 to 9 characterized by precipitating the supernatant liquid of the culture in the serum-free medium with ammonium sulfate and submitting the material to gel filtration with Sephacryl S-200, collecting the portion not absorbed by Blue Sepharose and passing it further through DEAE Sephadex A-50, and if desired subjecting it to HPLC with TSK 6-3000 SWG and/or with TSK DEAE-3SW.

11. The method according to claims 2 to 10 characterized by obtaining monoclonal antibodies from the hybridoma between spleen cells and mouse myeloma cells such as P3-X63-Ag 8.653 by injecting the proteinaceous substance according to claim 1 into the skin of the mice together with an adjuvant and further performing the intensive immunity.

12. The method according to claims 2 to 11 characterized by allowing the monoclonal antibodies to bond with a suitable carrier for further purification or quantitative determination.

# FIG.1

TSK-DEAE 3SW
7.5 ID× 75mm

NaCl conc.

1.0M

0.5M

0.15M  0.15

: OD₂₈₀nm

0.15

0.1

0.05

0

: ATF activity (×10⁴ units)

4

2

0

0    10    20    30    40    50

fraction number

1/4

0149751

# F I G . 2

3/4

0149751

# Photo . 1

# Photo . 3

Photo . 2